# EUROPÄISCHE PATENTANMELDUNG

(11) **EP 4 847 914 A1**
(43) Veröffentlichungstag der Anmeldung: **02.12.2026**
(21) Anmeldenummer: 25178808.9
(22) Anmeldetag: 26.05.2025
(51) Int. Cl.: G01N 27/22, G01N 33/24, G01N 33/38

(54) **VORRICHTUNG UND VERFAHREN ZUR MESSUNG DER FEUCHTIGKEIT EINES MATERIALS**

(71) Anmelder: Diener, Dominik, 57334 Bad Laasphe (DE)
(72) Erfinder: Diener, Dominik, 57334 Bad Laasphe (DE)
(74) Vertreter: Limbeck, Achim

(57) **Zusammenfassung**

Die vorliegende Erfindung betrifft eine Vorrichtung (1) zur Messung der Feuchtigkeit eines Materials, wobei die Vorrichtung (1) mindestens zwei Feuchtigkeitssensoren (10', 10") umfasst, die parallel ausgerichtet an einem zur Messung des Feuchtegehaltes in dem Material einbringbaren Messkörpers (11) der Vorrichtung (1) angeordnet sind, wobei die mindestens zwei Feuchtesensoren (10', 10") kapazitive Sensoren sind. Die erfindungsgemäße Vorrichtung (1) ist dadurch gekennzeichnet, dass die Feuchtigkeitssensoren (10', 10") länglich ausgebildet sind und sich zumindest bereichsweise und parallel zu einer gemeinsamen Achse (A) und an der Außenseite (110) des Messkörpers (11) erstrecken, wobei sich die Feuchtigkeitssensoren (10', 10") von einer ersten quer zur Längsachse (L) des Messkörpers (11) gerichteten Ebene (11.1) in Richtung einer zweiten quer zur Längsachse (L) des Messkörpers (11) gerichtete Ebene (11.2) des Messkörpers (11) in unterschiedlichen Höhen und/oder Längen und/oder zu unterschiedlichen quer zur Längsachse (L) des Messkörpers (11) gerichteten Ebenen (11.1, 11.2) am Messkörper (11) erstrecken und mindestens zwei Feuchtigkeitssensoren (10', 10") zumindest bereichsweise eine gemeinsame quer zur Längsachse (L) des Messkörpers (11) gerichtete Ebene (11.3) überlappen.

## Beschreibung

### Technisches Gebiet

Die vorliegende Erfindung betrifft eine Vorrichtung zur Messung der Feuchtigkeit eines Materials, insbesondere zur Bestimmung des Restfeuchtegehalts in Estrich und Beton, gemäß dem Oberbegriff des Patentanspruchs 1. Weiterhin betrifft die vorliegende Erfindung ein Verfahren zur Messung der Feuchtigkeit eines Materials mittels der erfindungsgemäßen Vorrichtung nach Patentanspruch 10.

### Stand der Technik

Aus dem Stand der Technik sind bereits verschiedenste Vorrichtungen und Methoden zur Messung der Feuchtigkeit eines Materials bekannt. Die sog. Darr-Methode sieht das Wiegen der Probe vor und nach der Zwangstrocknung im Ofen vor. Diese Methode kann nur im Labor durchgeführt werden und ist entsprechend aufwendig. Mittels der CM-Messung wird die chemische Reaktion mit Carbid zum Druckaufbau ermittelt. Die KRL-Methode betrifft die Messung des Anstiegs der Ausdunstung der Restfeuchte, bei dem die Gleichgewichtsfeuchte aus der Luft in einem geschlossenen System gemessen wird. Allen Verfahren ist gemein, dass bei diesen Messmethoden der Boden beschädigt wird und anschließend wieder verschlossen werden muss.

Die Ultraschallmessung sieht die Ermittlung der Beeinflussung des Laufs der Schallwellen im Boden vor. Die Oberflächen-Messung via Dialektrikum erfolgt kapazitiv. Die Ultraschallmessung und die Messung über Dialektrikum sind zwar preiswert und schnell in ihrer Durchführung, aber nur in wenige Zentimeter Tiefe des Untergrunds möglich sowie beeinflussbar durch verschiedene externen Faktoren und mithin ungenau.

Die Messung der Umgebungsfeuchtigkeit ist ebenfalls sehr ungenau und von zu vielen externen Einflüssen abhängig, welche das Ergebnis verfälschen können.

Darüber hinaus Messungen über die Lichtbrechung, elektrische Leitwiderstände und über den Einbau zweier Masse-Elektroden bekannt, in denen Strom wandert. Solche Methoden sind allerdings nur bedingt nutzbar, da die Messergebnisse ebenfalls zu ungenau sind und sich durch den Stromfluss im nassen Medium schnell abnutzen.

### Darstellung der Erfindung

Der vorliegenden Erfindung liegt die Aufgabe zugrunde, die vorgenannten Nachteile zu beseitigen und eine Vorrichtung zu schaffen, die es erlaubt, die Trocknung in Beton und Estrich zerstörungsfrei und ohne Einwirkung von außen zu überwachen und die Belegereife zu übermitteln.

Erfindungsgemäß wird die voranstehende Aufgabe gemäß dem Oberbegriff des Anspruchs 1 in Verbindung mit den kennzeichnenden Merkmalen gelöst. Vorteilhafte Ausgestaltungen und Weiterbildungen der erfindungsgemäßen Vorrichtung sind in den abhängigen Unteransprüchen angegeben.

Die erfindungsgemäße Vorrichtung umfasst zunächst mindestens zwei Feuchtigkeitssensoren, die parallel ausgerichtet an einem zur Messung des Feuchtegehaltes in dem Material einbringbaren Messkörpers der Vorrichtung angeordnet sind, wobei die mindestens zwei Feuchtesensoren kapazitive Sensoren sind.

Erfindungsgemäß ist die vorliegende Vorrichtung dadurch gekennzeichnet, dass die Feuchtigkeitssensoren länglich ausgebildet sind und sich zumindest bereichsweise und parallel zu einer gemeinsamen Achse und an der Außenseite des Messkörpers erstrecken, wobei sich die Feuchtigkeitssensoren von einer ersten quer zur Längsachse des Messkörpers gerichteten Ebene in Richtung einer zweiten quer zur Längsachse des Messkörpers gerichtete Ebene des Messkörpers in unterschiedlichen Höhen und/oder Längen und/oder zu unterschiedlichen quer zur Längsachse des Messkörpers gerichteten Ebenen am Messkörper erstrecken und mindestens zwei Feuchtigkeitssensoren zumindest bereichsweise eine gemeinsame quer zur Längsachse des Messkörpers gerichtete Ebene überlappen.

Durch die leichte Überlagerung der Feuchtigkeitssensoren ist gewährleistet, dass zum einen eine möglichst große Messfläche vorliegt, zum anderen kann die Vorrichtung dadurch erkennen, an welcher Stelle er gekürzt (geschnitten) wurde, wie dies nachstehend im Rahmen des Verfahrens näher beschrieben wird.

Die erfindungsgemäße Vorrichtung misst die Kapazität im zu messenden Material. Im zu messenden Material liegen zwei verschiedene Werte vor. Zum einen die fixe und unveränderliche Kapazität des Betons, Estrichs, Sand Zuschlagstoffe. Zum anderen die Variable in Form von Wasser. Letzteres wird in den ersten Tagen durch eine chemische Abbindung eingelagert / verdrängt und im zweiten Schritt stellt sich über den Austausch mit der im Raum vorliegenden Luft nach und nach eine Ausgleichsfeuchte zur Umgebung her.

Um eine Zeichnung der Messwerte beispielweise durch Labor-Messung zu ermöglichen, wird aus den Messwerten ein Mittelwert gebildet von den Ebenen, welche Kontakt mit dem Estrich haben. Ebenen, welche außerhalb des Estrichs angeordnet sind, werden sowohl bei der Labormessung als auch bei der Baustellenmessung heraus gerechnet. Dies erfolgt über das Abschneiden des Messkörpers über die integrierte Elektronik. Der ermittelte Mittelwert wird über eine anerkannte Messmethode (Darr-Methode) kalibriert. In einem Beispiel sind drei Ebenen im Estrich, die vierte Ebene wird bei der Mittelwert-Ermittlung nicht berücksichtigt. Um eine möglichst hohe Genauigkeit zu ermitteln, sollte für jede Ebene eine eigene Messung durchgeführt werden.

Bei abweichenden Werten mehrerer an unterschiedlichen, sich gegenüberliegenden und/oder aneinander angrenzenden Seiten des Messkörpers angeordneten Feuchtigkeitssensoren gleicher Länge wird vorzugsweise die Abweichung der durch diese Feuchtigkeitssensoren ermittelten Werte in der Weise kompensiert, dass als Ausgangsmesswert der höhere oder der niedrigere Wert zur Ermittlung des Feuchtegehalts verwendet wird.

Die erfindungsgemäße Vorrichtung besteht vorzugsweise im Kern aus drei wesentlichen Komponenten. 1. dem Messkörper, 2. einer steckbaren Sendeeinheit per Funk, wobei die Sendeeinheit vorzugsweise über eine wiederaufladbare Batterie, ein Modem zur Datenübertragung sowie einem (Auswerte-)Chip verfügt, wobei an der Unterseite eine Stiftleiste bzw. an der Außenseite ein Federkontakt angebracht sein kann, wodurch eine Verbindung zum Messkörper hergestellt wird und die Einheit zusätzlich geladen und programmiert werden kann. Alternativ kann eine steckbare Ausleseeinheit per Kabel vorgesehen sein, wie zuvor jedoch ohne Batterie und Modem. Die Stromversorgung erfolgt kabelgebunden, das Auslesen der Daten beispielsweise über eine serielle Schnittstelle.

Die Messung der Kapazität erfolgt über den Messkörper, welcher vorzugsweise eine Vielzahl an beispielsweise als Messstreifen ausgebildeten Feuchtigkeitssensoren in verschiedenen Längen (über die Höhe des Sensors) verfügt, um zum einen Störsignale wie z.B. wasserführende Rohre oder Kabel zu ermitteln, aber auch um den Messkörper in der Höhe flexibel anzupassen. Bei den Feuchtigkeitssensoren handelt es sich vorzugsweise um eine Polycarbonat-Folie in welche einzelne leitfähige Streifen (aus z.B. Kupfer oder Leitsilber) in unterschiedlichen Längen eingebracht sind. Durch die beidseitige Laminierung sind die leitfähigen Streifen vollkommen von dem zum messenden Material durch eine definierte Stärke und Eigenschaft getrennt. Über einen außen angebrachten weiteren leitfähigen Streifen wird die Masse-Verbindung zum messenden Material hergestellt. Es wird hier ein Kondensator aufgebaut.

Der Messkörper wird im Neubau beispielsweise in rechteckiger Form ausgeführt, um das Abziehen mit Glätte-Kellen zu ermöglichen. Im Sanierungsbau kann der Messkörper rund ausgeführt sein, um nach einer Kernbohrung in den Boden eingesteckt zu werden. In beiden Fällen ist der Effekt genauer je näher er an dem zu messenden Objekt anliegt.

Der Messkörper wird nach dem Aushärten des zu messenden Materials mit einem glatten Werkzeug (z.B. Oszilationsschneider) an der Oberkante abgetrennt. Dadurch wird eine Stolperkante auf das geringste Maß reduziert. Die Abdeckkappe wird als Staubschutz wieder montiert.

Wenn mehr als zwei Feuchtigkeitssensoren unterschiedlicher Höhen unbeschädigt sind, kann zusätzlich der Trocknungsverlauf genauer in der Höhe visualisiert werden. Hier wird ermittelt ob das Material eher weiter oben oder weiter unten eine andere Feuchtigkeit aufweist.

Zwischen den Feuchtigkeitssensoren und dem offenen Kontakt wird ein elektrisches Feld aufgebaut, dessen Kapazität sich aufgrund der Änderung der Dielektrizitätskonstante des Materials zwischen den Elektroden ändert. Durch die Sättigung des Estrichs / Beton mit Wasser verhält sich dies identisch wie reines Wasser wodurch die Messhöhe ermittelt wird. Dies ist durch die bekannte Dielektrizitätskonstante von Wasser möglich.

Der ermittelte und bereinigte Wert wird in Verbindung mit der Höhe und unter Anpassung durch der Störkapazitität in das Verhältnis einer Referenzmessprobe auf den korrekten Restfeuchte-Wert angepasst. Die Referenzmessung erfolgt im Labor über die selbe Messtechnik und wird mit einer CM-Messung, besser einer Darr-Messung, festgesetzt.

Temperatur-Kompensation: Durch innere (Heizung) und äußere (Raumheizung / Sonneneinstrahlung) Temperatureinwirkung driftet die Temperatur nach oben oder unten. Bis zu einem gewissen Punkt sinkt der Messwert. Dies ist auf die Verdrängung der Feuchtigkeit aus dem Kunststoff zurückzuführen. Ab einer gewissen Temperatur geht das enthaltene Wasser von einem flüssigen in den gasförmigen Zustand über, was den Messwert um ein Vielfaches nach oben treibt. Um diese Schwankungen zu eliminieren, kann bei jeder Messung die Temperatur zur Kompensation ermittelt werden.

Durch einen vorzugsweise vorgesehenen - und beispielsweise in der Sendeeinheit verbauten - Temperatur-Sensor kann ferner zusätzlich die exotherme Abbindung oder künstlich zugeführte Wärme (zum Beispiel Fußbodenheizung, Heizstrahler, Sonnenlicht) protokolliert und ausgewertet werden. Hierzu sind die Temperaturverlaufskurven in Verbindung für Zement weithin bekannt oder können durch einfache Labormessungen ermittelt werden. Der chemische Abbindeprozess stoppt, sobald nicht mehr genügend Wasser vorhanden ist. Dadurch endet die exotherme Phase und die weitere Trocknung erfolgt über Verdunstung. An diesem Punkt ist die genaue Restmenge an Wasser im zum messenden Material auswertebar. In Verbindung mit den zuvor im Labor ermittelten Endwerten kann eine exakte Trocknungskurve ermittelt werden.

Verfahrensgemäß wird der Messkörper der erfindungsgemäßen Vorrichtung in seiner Längsrichtung lotrecht in ein zu untersuchendes Material eingebracht, um den darin befindlichen Feuchtegehalt kapazitiv zu erfassen.

Dabei kann es vorteilhaft sein, wenn nach dem Einbringen des Messkörpers in das Material der Messkörper nach dem Aushärten des in seiner Längsrichtung gekürzt wird, um einen nach dem Einbringen in das Material etwaigen außerhalb des Materials liegenden Bereich des Messkörpers zu beseitigen.

Nach dem Kürzen des Messkörpers wird die Abdeckung des Messkörpers montiert wird.

### Kurzbeschreibung der Zeichnungen

Weitere Ziele, Merkmale, Vorteile und Anwendungsmöglichkeiten der erfindungsgemäßen Vorrichtung ergeben sich aus der nachfolgenden Beschreibung eines Ausführungsbeispiels anhand der Zeichnungen.

In den Zeichnungen zeigen
Fig. 1 die erfindungsgemäße Vorrichtung in einer vorteilhaften Ausführungsform in der Seitenansicht;
Fig. 2 die erfindungsgemäße Vorrichtung wie in Fig. 1 in perspektivischer Ansicht;
Fig. 3 die Bodenplatte der erfindungsgemäßen Vorrichtung in perspektivischer Ansicht;
Fig. 4 die erfindungsgemäße Vorrichtung mit Bodenplatte und Abdeckung in perspektivischer Ansicht.

### Ausführung der Erfindung

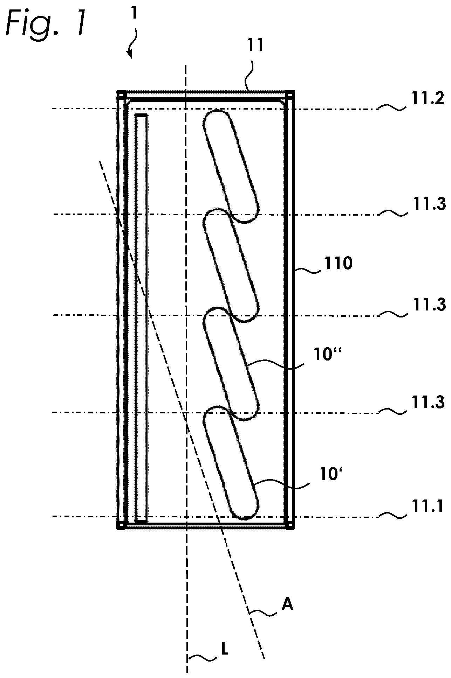

Wie aus Fig. 1 ersichtlich, umfasst die erfindungsgemäße Vorrichtung mehrere parallel und schräg an dem Messkörper 11 angeordnete Feuchtigkeitssensoren 10', 10'', wobei sich die Feuchtigkeitssensoren 10', 10'' von einer ersten quer zur Längsachse L des Messkörpers 11 gerichteten Ebene 11.1 in Richtung einer zweiten quer zur Längsachse L des Messkörpers 11 gerichtete Ebene 11.2 des Messkörpers 11 in unterschiedlichen Höhen und zu unterschiedlichen quer zur Längsachse L des Messkörpers 11 gerichteten Ebenen 11.2, 11.3 am Messkörper 11 erstrecken. Sämtliche Feuchtigkeitssensoren 10', 10'' überlappen jeweils zumindest bereichsweise eine gemeinsame quer zur Längsachse L des Messkörpers 11 gerichtete Ebene 11.3, hier in Fig. 1 dargestellt entsprechend mehrere Ebenen 11.3.

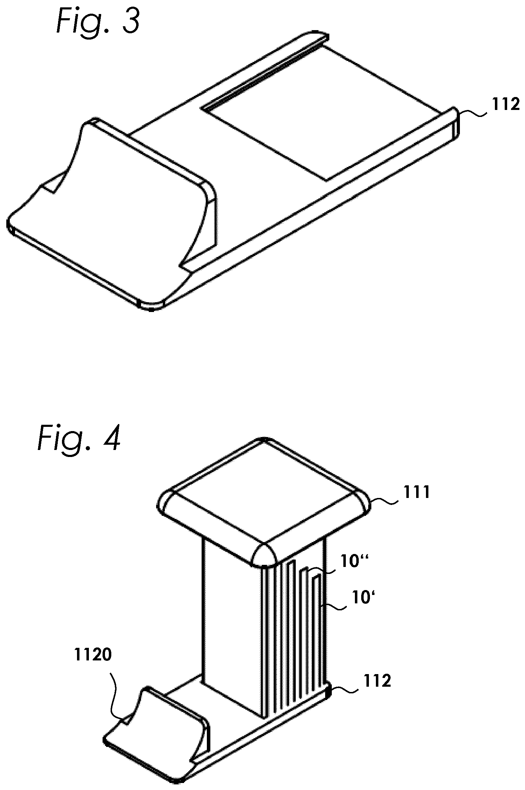

Wie aus Fig. 4 ersichtlich, können sich mehrere Feuchtigkeitssensoren 10', 10'' auch in Längsrichtung L des Messkörpers 11 in unterschiedlichen Längen und/oder Höhen parallel beabstandet erstrecken.

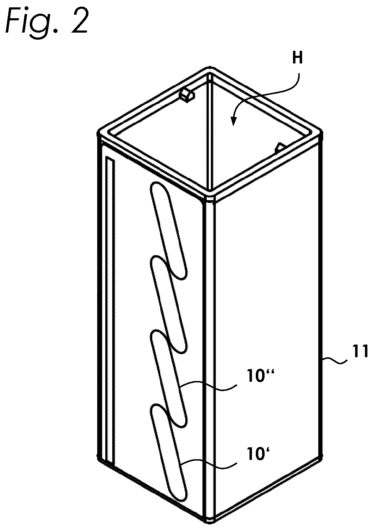

Wie aus Fig. 2 ersichtlich, ist der Messkörper 11 vorzugsweise ein Hohlkörper, wobei die Feuchtigkeitssensoren 10', 10'' eine Leiterbahn und/oder einen Leiterkontakt umfassen, welche/r innerhalb oder unterhalb des vorzugsweise unterseitg verschlossenen Hohlkörpers enden und dort mit einer Schnittstelle und/oder einer Sendeeinheit verbunden sind.

Unterseitig des Hohlkörpers ist vorzugsweise ein Bodenteil 112 vorgesehen, mittels dem der Hohlkörper bzw. der Messkörper 11 fixiert werden kann. Gemäß Fig. 4 umfasst der Messkörper 11 weiterhin vorzugsweise eine zumindest einseitige (obere) Abdeckung 111.

An dem Messkörper 11 ist weiterhin vorzugsweise mindestens eine vorteilhafterweise durch das untere Bodenteil 112 gebildete Abkantung 1120 nach außen vorgesehen, um die Vorrichtung 1 im beispielsweise erhärteten Fließestrich unterhalb der Fußbodenheizung festzuklemmen.

In einer besonders vorteilhaften Ausführungsform der Erfindung erfolgt die Kontaktierung der Sendeeinheit über eine Buchsenleiste, welcher mit einer Steckerleiste der Sendeeinheit kontaktiert wird, wobei alternativ an der Sendeeinheit auch entsprechend Federkontakte zur Kontaktierung vorgesehen sein können.

Ausführung 1: Die Kontaktierung erfolgt über eine Stiftleiste in der Sendeeinheit und kontaktiert auf eine Buchsenleiste in dem Messkörper 11. Um mit dem Mobilfunk- und RFID-Signal aus dem Messkörper 11 heraus zu kommen, wird vorzugsweise eine innenliegende und wie der Sensor selbst abschneidbare Antennenleitung vertikal nach oben geführt. Das Antennensignal wird im oberen Bereich über einen Federkontakt auf die Antenne im Deckel übertragen.

Ausführung 2: Die Kontaktierung erfolgt über Federkontakte, die in der Sendeeinheit über eine oder mehr Seiten nach außen abführen und auf die Kontaktfläche kontaktieren, welche auf der Innenseite des Messkörpers 11 vertikal verlaufen.

Das Bodenteil 112 ist vorzugsweise ansteckbar an dem Messkörper 11 angeordnet und wird benötigt, um beim Fließestrich ein Aufschwimmen zu verhindern. Das Bodenteil 112 kann unter die vorhandene Fußbodenheizung gesteckt werden. Dadurch wird ein definierter Abstand zu dem Wasserführenden System hergestellt, wodurch eine definierte Beeinflussung des Sensors stattfindet. Wenn keine Fußbodenheizung vorhanden ist, muss das Bodenteil 112 mit dem Untergrund verklebt werden.

Die Vorrichtung 1 umfasst vorzugsweise einen RFID-Chip, welcher per Funk ausgelesen werden kann. Die RFID-Antenne befindet sich beispielsweise in der Abdeckung 111 des Messkörpers 11. Die von der Vorrichtung 1 ermittelten Daten werden regelmäßig von der Sendeeinheit in den RFID-Chip geschrieben und können mit geeigneten Endgeräten ausgelesen werden. Der RFID-Chip kann unter anderem folgende Daten umfassen: Einbaudatum, Art des Materials, Aufbauhöhe, Trocknungsdauer, Heizkurve, ID des Estrich-Vorgangs.

### Liste der Bezugsziffern

- 1: Vorrichtung
- 10', 10": Feuchtigkeitssensoren
- 11: Messkörper
- 11.1, 11.2: Ebenen
- 11.3: überlappte Ebenen
- 110: Außenseite des Messkörpers
- 111: (obere) Abdeckung
- 112: (unteres) Bodenteil
- 1120: Abkantung
- A: Achse
- H: Hohlraum
- L: Längsachse des Messkörpers

## Patentansprüche

1. Vorrichtung (1) zur Messung der Feuchtigkeit eines Materials, wobei die Vorrichtung (1) mindestens zwei Feuchtigkeitssensoren (10', 10'') umfasst, die parallel ausgerichtet an einem zur Messung des Feuchtegehaltes in dem Material einbringbaren Messkörpers (11) der Vorrichtung (1) angeordnet sind, wobei die mindestens zwei Feuchtesensoren (10', 10'') kapazitive Sensoren sind,
**dadurch gekennzeichnet, dass**
a) die Feuchtigkeitssensoren (10', 10'') länglich ausgebildet sind und sich zumindest bereichsweise und parallel zu einer gemeinsamen Achse (A) und an der Außenseite (110) des Messkörpers (11) erstrecken, wobei
b) sich die Feuchtigkeitssensoren (10', 10'') von einer ersten quer zur Längsachse (L) des Messkörpers (11) gerichteten Ebene (11.1) in Richtung einer zweiten quer zur Längsachse (L) des Messkörpers (11) gerichtete Ebene (11.2) des Messkörpers (11) in unterschiedlichen Höhen und/oder Längen und/oder zu unterschiedlichen quer zur Längsachse (L) des Messkörpers (11) gerichteten Ebenen (11.1, 11.2) am Messkörper (11) erstrecken und
c) mindestens zwei Feuchtigkeitssensoren (10', 10'') zumindest bereichsweise eine gemeinsame quer zur Längsachse (L) des Messkörpers (11) gerichtete Ebene (11.3) überlappen.

2. Vorrichtung (1) nach Anspruch 1,
**dadurch gekennzeichnet, dass**
sie mindestens einen Temperatursensor umfasst.

3. Vorrichtung (1) nach Anspruch 1 oder 2,
**dadurch gekennzeichnet, dass**
sie eine funkbasierte Sendeeinheit umfasst, mittels der die durch die Feuchtigkeitssensoren (10', 10'') und/oder den mindestens einen Temperatursensor ermittelten Feuchtigkeits- und/oder Temperaturmesswerte an eine Empfängereinheit übertragbar sind.

4. Vorrichtung (1) nach einem oder mehreren der vorangegangenen Ansprüche,
**dadurch gekennzeichnet, dass**
sie eine kabelgebundene Schnittstelle umfasst, mittels der die durch die Feuchtigkeitssensoren (10', 10'') und/oder den mindestens einen Temperatursensor ermittelten Feuchtigkeits- und/oder Temperaturmesswerte an eine Empfängereinheit übertragbar sind.

5. Vorrichtung (1) nach einem oder mehreren der vorangegangenen Ansprüche,
**dadurch gekennzeichnet, dass**
die Feuchtigkeitssensoren (10', 10'') aus einzelnen elektrisch leitfähige Messstreifen gebildet sind, die in eine oder mehrere Folien und/oder in das Material des Messkörpers (1) eingebracht sind.

6. Vorrichtung (1) nach Anspruch 5,
**dadurch gekennzeichnet, dass**
die Folie aus Polycarbonat besteht.

7. Vorrichtung (1) nach einem oder mehreren der vorangegangenen Ansprüche,
**dadurch gekennzeichnet, dass**
der Messkörper (11) ein Hohlkörper ist, wobei die Feuchtigkeitssensoren (10', 10'') eine Leiterbahn und/oder einen Leiterkontakt umfassen, welche/r unter- und/oder innerhalb des Hohlkörpers des Messkörpers (11) enden und dort mit einer Schnittstelle oder einer Sendeeinheit verbunden sind.

8. Vorrichtung (1) nach Anspruch 7,
**dadurch gekennzeichnet, dass**
der Messkörper (11) eine zumindest einseitige Abdeckung (111) und/oder ein Bodenteil (112) aufweist.

9. Vorrichtung (1) nach einem oder mehreren der vorangegangenen Ansprüche,
**dadurch gekennzeichnet, dass**
an dem Messkörper (11) mindestens eine Abkantung (1120) nach außen vorgesehen ist, um die Vorrichtung (1) im noch nicht erhärteten Untergund festzuklemmen.

10. Verfahren zur Messung der Feuchtigkeit eines Materials mittels einer Vorrichtung (1) nach einem oder mehreren der vorangegangenen Ansprüche,
**dadurch gekennzeichnet, dass**
der Messkörper (11) der Vorrichtung (1) in seiner Längsrichtung (L) lotrecht oder waagerecht in ein zu untersuchendes Material eingebracht wird, um den darin befindlichen Feuchtegehalt kapazitiv zu erfassen.

11. Verfahren Anspruch 10,
**dadurch gekennzeichnet, dass**
nach dem Einbringen des Messkörpers (11) in das Material der Messkörper (11) nach dem Aushärten des in seiner Längsrichtung (L) gekürzt wird, um einen nach dem Einbringen in das Material etwaigen außerhalb des Materials liegenden Bereich (11.3) des Messkörpers (11) zu beseitigen.

12. Verfahren nach Anspruch 10 und/oder 11,
**dadurch gekennzeichnet, dass**
nach dem Kürzen des Messkörpers (11) die Abdeckung (111) des Messkörpers (11) montiert wird.

13. Verfahren nach einem oder mehreren der vorangegangenen Ansprüche,
**dadurch gekennzeichnet, dass**
durch die mehreren Feuchtigkeitssensoren (10', 10'') der Feuchtegehalt des Materials (4) in unterschiedlichen Tiefen im Material erfasst wird.

14. Verfahren nach einem oder mehreren der vorangegangenen Ansprüche,
**dadurch gekennzeichnet, dass**
mittels des Temperatursensors die exotherme Abbindung oder künstlich zugeführte Wärme des Materials ermittelt wird.

15. Verfahren nach einem oder mehreren der vorangegangenen Ansprüche 10 bis 14,
**dadurch gekennzeichnet, dass**
bei abweichenden Werten mehrerer an unterschiedlichen, sich gegenüberliegenden und/oder aneinander angrenzenden Seiten des Messkörpers (11) angeordneten Feuchtigkeitssensoren (10', 10'') gleicher Länge die Abweichung der durch diese Feuchtigkeitssensoren (10', 10'') ermittelten Werte in der Weise kompensiert werden, dass als Ausgangsmesswert der höhere oder der niedrigere Wert zur Ermittlung des Feuchtegehalts verwendet wird.
